Europäisches Patentamt

European Patent Office

Office européen des brevets

(11) Veröffentlichungsnummer : **0 328 957 B1**

(12)

# EUROPÄISCHE PATENTSCHRIFT

(45) Veröffentlichungstag der Patentschrift :
**29.07.92 Patentblatt 92/31**

(51) Int. Cl.$^5$ : **A61K 45/06, A61K 37/54,
A61K 37/547, // (A61K37/54,
35:62), (A61K37/547, 35:62)**

(21) Anmeldenummer : **89101849.1**

(22) Anmeldetag : **03.02.89**

(54) Mischung aus einer thrombolytisch wirkenden und einer antithrombotischen Substanz.

Die Akte enthält technische Angaben, die nach dem Eingang der Anmeldung eingereicht wurden und die nicht in dieserPatentschrift enthalten sind.

(30) Priorität : **13.02.88 DE 3804600**

(43) Veröffentlichungstag der Anmeldung :
**23.08.89 Patentblatt 89/34**

(45) Bekanntmachung des Hinweises auf die Patenterteilung :
**29.07.92 Patentblatt 92/31**

(84) Benannte Vertragsstaaten :
**AT BE CH DE ES FR GB IT LI NL**

(56) Entgegenhaltungen :
**EP-A- 181 267**

(56) Entgegenhaltungen :
**CHEMICAL ABSTRACTS, Band 108, 1988, Seite 46, Zusammenfassung Nr. 68585y, Columbus, Ohio, US; J.M. STASSEN et al.:"Potentiation by heparin fragments of thrombolysis induced with human tissue-type plasminogen activator or human single-chai-nurokinase-type plasminogen activator", & THROMB. HAEMOSTASIS 1987, 58(3), 947-50 IDEM
THROMBOSIS HAEMOSTATIS, Band 47, 1982, Seiten 226-229; F. MARKWARDT et al.:
"Pharmacological studies on the antithrombo-ticaction of hirudin in experimental animals" Stassen J.M. et al. Thrombosis Maemostasis, 58 (3), 947-950, 1987**

(73) Patentinhaber : **BASF Aktiengesellschaft
Carl-Bosch-Strasse 38
W-6700 Ludwigshafen (DE)**

(72) Erfinder : **Eschenfelder, Volker, Dr.
Blumenstrasse 57
W-6908 Wiesloch (DE)**
Erfinder : **Schmied, Bernhard, Dr.
Taunusstrasse 20
W-6710 Frankenthal (DE)**
Erfinder : **Ruebsamen, Klaus, Dr.
Erschigweg 19
W-6730 Neustadt (DE)**

EP 0 328 957 B1

**Beschreibung**

Die vorliegende Erfindung betrifft eine Mischung aus einer thrombolytisch wirkenden und einer antithrombotischen Substanz zur Bekämpfung thrombotisch bedingter Gefäßerkrankungen.

Es ist bereits bekannt, daß t-PA (tissue plasminogen activator) durch Aktivierung von Plasminogen zur Auflösung von Fibrin und somit zur Reperfusion thrombosierter Gefäße führt (EP-OS 41 766). Weiter ist bekannt, daß durch gleichzeitige Applikation einer selektiven antithrombotischen Substanz (= Thrombinantagonist) wie MCI 9038 (= (2R,4R)-4-methyl-1-[N²-[(3-methyl-1,2,3,4-tetrahydro-8-quinolinyl)-sulfonyl]-L-arginyl]-2-piperidine carboxylic acid) oder Heparin die zur Lyse eines thrombosierten Blutgefäßes erforderliche Zeit erheblich verkürzt werden kann, indem die Neuablagerung von Fibrin am bestehenden Thrombus verhindert wird (Thrombosis & Haemostasis 56 (1986) 28).

Gegenstand der Erfindung ist eine Mischung aus einer thrombolytisch wirkenden Substanz und einer antithrombotischen Substanz, dadurch gekennzeichnet, daß die thrombolytisch wirkende Substanz t-PA und die antithrombotische Substanz Hirudin ist.

Die Bezeichnung t-PA bedeutet t-PA selbst sowie die sich davon durch Substitution, Modifikation und/oder Deletion einer oder mehrerer Aminosäuren oder Kettenverkürzung ableitenden Polypeptide mit t-PA-Wirkung. Diese Substanzen können in glykolisierter oder nichtglykolisierter Form vorliegen. Solche Polypeptide sind beispielsweise in folgenden Patenten bzw. Patentanmeldungen genannt:

| | | |
|---|---|---|
| DE | 3 011 956 | EP 93 619 |
| | 3 240 174 | 124 613 |
| | 3 502 760 | 152 736 |
| | 3 537 176 | 155 388 |
| GB | 2 173 804 | 174 835 |
| | | 178 105 |
| WO | 84/01786 | 184.363 |
| | 86/01538 | 196.920 |
| | | 198.015 |

Die Bezeichnung Hirudin schließt die sich davon durch Substitution, Modifikation und/oder Deletion einer oder mehrerer Aminosäuren oder Kettenverkürzung ableitende Polypeptide mit Hirudin-Wirkung ein. Die Substanzen können in glykosilierter oder nichtglykosilierter bzw. in sulfatierter und nicht-sulfatierter Form vorliegen. Solche Substanzen sind beispielsweise in folgenden Schutzrechten beschrieben:

| | | |
|---|---|---|
| DE | 3 445 517 | EP 158 986 |
| | 3 526 995 | 168 342 |
| FR | 8 404 755 | 171 024 |
| | 8 506 672 | 200 655 |
| WO | 85/04418 | |
| | 86/06406 | |

Die Bestandteile der Mischung können auch in Form von Säureadddtionssalzen vorliegen. Als Säureadditionssalze kommen insbesondere physiologisch verträgliche Salze mit üblichen, therapeutisch anwendbaren Säuren in Betracht; als anorganische Säuren sind die Halogenwasserstoffsäuren, wie die Chlorwasserstoffsäure, sowie Schwefelsäure und Phosphor-bzw. Pyrophosphorsäure zu nennen; als organische Säuren sind in erster Linie Sulfonsäuren, wie die Benzol- oder p-Toluolsulfonsäure oder Niederalkansulfonsäuren, wie Methansulfonsäure, sowie Carbonsäuren, wie Essigsäure, Milchsäure, Palmitin- und Stearinsäure, Äpfelsäure, Weinsäure, Ascorbinsäure und Citronensäure, geeignet. Da die Mischungsbestandteile auch Aminosäurereste mit freien Carboxylgruppen enthalten, können sie auch als Metallsalz, insbesondere als Alkalimetall- oder Erdalkalimetallsalz, z.B. Natrium-, Kalium-, Calcium- oder Magnesiumsalz, oder auch als Ammoniumsalz, abgeleitet von Ammoniak oder einer physiologisch verträglichen, organischen stickstoffhaltigen Base, vorliegen. Sie können auch als inneres Salz vorliegen.

Das t-PA und das Hirudin liegen in der Mischung im Mengenverhältnis von 10:1 bis 500:1, vorzugsweise 50:1 bis 100:1 vor.

Die Mischung kann in Form einer Lösung intraarteriell, intravenös oder subkutan, vorzugsweise durch Infundieren appliziert werden. Die Lösung enthält neben den Wirkstoffen üblicherweise noch einen Puffer, z.B. einen Phosphatpuffer, der den pH-Wert zwischen etwa 3,5 und 7 halten soll, und ferner Natriumchlorid, Mannit oder Sorbit zur Einstellung der Isotonie. Sie können in gefriergetrockneter oder gelöster Form vorliegen und ein antibakteriell wirkendes Konservierungsmittel enthalten.

Mit Hilfe der neuen Kombination lassen sich Thrombosen schneller und sicherer auflösen als mit t-PA alleine. Das ist insofern überraschend als Hirudin keine thrombolytische Wirkung besitzt. Weiter kann durch die kombinierte Anwendung beider Substanzen die Häufigkeit einer Reocclusion nach Lyse eines bestehenden Thrombus deutlich reduziert werden. Die Mischung eignet sich daher zur Behandlung von Thrombosen jeglicher Art, wie Thrombophlebitis, Hämorrhoidalthrombose, Lungenembolie und Infarkt.

Pro Therapie wird dem Patienten eine solche Menge von der Mischung zugeführt, daß die applizierte Menge an t-PA ingesamt zwischen 50 und 100 mg pro Patient liegt.

Die Applikation ist in der Regel nach 2 Stunden beendet.

Beispiel A

Herstellung einer Lösung

1 g t-PA und 15 mg Hirudin (HV 2 gemäß EP 158 564) werden in 100 ml 0,1 M Natriumphosphatpuffer pH 6,0 gelöst. Die Lösung wird anschließend mit Natriumchlorid versetzt, so daß eine blutisotonische Lösung entsteht. Die Lösung wird steril filtriert und in 5 ml-Ampullen gefüllt.

Beispiel B

Nachweis der Wirkung

Narkotisierte Kaninchen werden auf einer thermostatisierten Unterlage in Rückenlage fixiert und nach Tracheotomie künstlich beatmet. In die A.carotis und V.jugularis werden zur Blutdruckmessung bzw. zur Infusion von t-PA PE-Katheter eingebunden. Ein weiterer Katheter zur Druckmessung wird in die A.femoralis dextra eingebunden. Danach werden die rechte und linke A.iliaca externa bis zur Einmündung in die Aorta abdominalis freipräpariert. Ein kurzes Segment der A.abdominalis wird vom umgebenden Gewebe befreit und mit einem elektromagnetischen Flußmeßkopf (Gould) versehen. Danach wird durch die linke A.iliaca mit Hilfe eines PE-Katheters eine Kupferspirale bis in die A.abdominalis vorgeschoben. Dort wird die Spirale nach Freisetzung aus dem Katheter vom Blutstrom erfaßt und in die A.iliaca dextra eingeschwemmt. Nach Entfernen des Katheters wird die A.iliaca sinistra abgebunden. Im Bereich der Spirale kommt es innerhalb kurzer Zeit zur Thrombusbildung. Zeitpunkt und Ausmaß des dadurch bedingten Verschlusses der A.femoralis werden durch den distal von der Spirale gemessenen Druckabfall und durch den verringerten Blutfluß in der A.abdominalis bestimmt.

30 min nach Verschluß des Gefäßes durch den Thrombus wird rt-PA als Bolus (80 µg/kg), gefolgt von einer Infusion von 8 µg/kg · min, intravenös appliziert. Gleichzeitig mit der Infusion von rt-PA wird r-Hirudin ebenfalls als Infusion verabreicht. Die t-PA-Infusion wird beendet nach Erreichen einer mindestens 10 min andauernden Reperfusion, spätestens aber nach 2 h. Die Infusion von Hirudin wird erst nach Abschluß der 3-stündigen Versuchsperiode beendet. Bei den Tieren, bei denen Reperfusion innerhalb von 2 h auftritt, wird nach Beendigung der t-PA-Infusion die Zeit bis zur Reocclusion bestimmt.

An einem durch eine Kupferspirale in der A.iliaca induzierten Thrombus wurde der Einfluß von r-Hirudin auf die thrombolytische Wirkung von rekombinantem t-PA bestimmt. Weiterhin wurde untersucht, ob und in welchem Umfang r-Hirudin nach erfolgreicher Lyse den Wiederverschluß des Gefäßes verhindern kann.

Nach Positionierung der Kupferspirale in der A.iliaca kam es im Mittel nach 8,4 ± 0,5 min (n = 24) zu einem vollständigen, stabilen Gefäßverschluß. Herzfrequenz und mittlerer Blutdruck wurden durch diese Manipulation nicht nennenswert beeinflußt.

Bei 7 von 12 Tieren führte die Infusion von rt-PA im Mittel nach 75,3 ± 6,8 min zu Rekanalisation des verschlossenen Blutgefäßes (Tab. 1). Bei 5 Tieren wurde die Infusion nach 2 h erfolglos abgebrochen. Diese Versuche wurden nicht in die weitere Auswertung einbezogen.

Mit rt-PA in gleicher Dosierung aber in Kombination mit r-Hirudin wurde der Thrombus bei 9 von 12 Tieren vollständig aufgelöst. Die mittlere Zeit bis zur Reperfusion betrug 69,1 ± 4,2 min.

Nach erfolgreicher Lyse kam es nach Absetzen der t-PA-Infusion bei allen Tieren zu Reocclusion des eröffneten Blutgefäßes nach im Mittel 21,9 ± 4,2 min. Erste Anzeichen der beginnenden Reocclusion sind zyklische Flußvariationen (CFV), die bereits wenige Minuten nach Infusionsende auftraten (Tab. 1).

Mit r-Hirudin in Kombination mit rt-PA konnte die Reocclusion nach erfolgreicher Lyse bei 7 Tieren völlig verhindert werden, bei 2 Tieren trat eine Reocclusion erst nach 35 bzw. 45 min auf. Zyklische Flußvariationen wurden nicht beobachtet.

Die erhaltenen Daten zeigen:

– Durch Kombination von t-PA mit r-Hirudin konnte die Häufigkeit, mit der eine Reperfusion verschlossener Blutgefäße erzielt wurde, von 58 auf 75 % gesteigert werden.

– r-Hirudin führte zu einem signifikanten Rückgang der Reocclusionshäufigkeit nach erfolgreicher Lyse.

– r-Hirudin verhinderte das Auftreten der auf die Bildung lokaler Thrombozytenaggregate zurückzuführenden zyklischen Blutflußvariationen vollständig.

Tabelle 1

Thrombolyse und Reocclusionszeit nach intravenöser Applikation von rt-PA oder rt-PA in Kombination mit r-Hirudin bei Kaninchen mit einem durch eine Kupferspirale induzierten Thrombus in der A.iliaca.

| Tier-Nr. | Zeit bis Reperfusion (min) | Reocclusionszeit (min) | Anzahl zyklischer Flußvariationen | Blutfluß in A. iliaca (ml/min) | | |
|---|---|---|---|---|---|---|
| | | | | Ausgangswert | nach Kupferspirale | nach Reperfusion |
| **rt-PA** | | | | | | |
| 1 | 56 | 8 | 5 | 51 | 20 | 27 |
| 2 | * | - | - | 98 | 24 | - |
| 3 | 79 | 36 | 4 | 67 | 29 | 12 |
| 4 | * | - | - | 111 | 9 | - |
| 5 | 79 | 10 | 6 | 99 | 12 | 11 |
| 6 | 111 | 36 | 7 | 98 | 21 | 27 |
| 7 | 72 | 19 | 0 | 90 | 30 | 12 |
| 8 | ´70 | 21 | 0 | 67 | 48 | 48 |
| 9 | * | - | - | 32 | 14 | - |
| 10 | * | - | - | 90 | 84 | - |
| 11 | 60 | 23 | 0 | 27 | 13 | 17 |
| 12 | * | - | - | 75 | 66 | - |
| x±SEM | 75,3±6,8 | 21,9±4,2 | 3,1±1,2 | 75,3±7,9 | 30,8±6,8 | 22,0±5,1 |
| **rt-PA + Hirudin (0,1 mg/kg · h)** | | | | | | |
| 13 | * | - | - | 83 | 8 | - |
| 14 | 50 | 35 | 0 | 66 | 57 | 53 |
| 15 | 57 | > 60 | 0 | 150 | 84 | 21 |
| 16 | 49 | > 60 | 0 | 83 | 23 | 17 |
| 17 | 77 | > 60 | 0 | 30 | 5 | 8 |
| 18 | * | - | - | 27 | 0 | - |
| 19 | * | - | - | 40 | 0 | - |
| 20 | 80 | > 60 | 0 | 40 | 17 | 17 |
| 21 | 95 | > 60 | 0 | 33 | 20 | 25 |
| 22 | 85 | > 60 | 0 | 36 | 10 | 8 |
| 23 | 47 | > 60 | 0 | 33 | 10 | 10 |
| 24 | 22 | 45 | 0 | 72 | 21 | 15 |
| x±SEM | 69,1±4,2 | | | 57,8±10,3 | 22,7±9,0 | 19,3±4,6 |

\* keine Reperfusion innerhalb 120 min

**Patentansprüche**

**Patentansprüche für folgende Vertragsstaaten : AT, BE, CH, DE, FR, GB, IT, LI, NL**

1. Mischung aus einer thrombolytisch wirkenden Substanz und einer antithrombotischen Substanz, dadurch gekennzeichnet, daß die thrombolytisch wirkende Substanz t-PA und die antithrombotische Substanz Hirudin ist.

2. Mischung gemäß Anspruch 1, dadurch gekennzeichnet, daß das Verhältnis von t-PA zu Hirudin 10 : 1 bis 500 : 1 beträgt.

**Patentansprüche für folgenden Vertragssaat : ES**

1. Verfahren zur Herstellung einer Wirkstofflösung, dadurch gekennzeichnet, daß man t-PA und Hirudin im Mengenverhältnis von 10:1 bis 500:1 in einem Puffer bei pH 3,5 und 7 löst und die Lösung isotonisch einstellt.

**Claims**

**Claims for the following Contracting States : AT, BE, CH, DE, FR, GB, IT, LI, NL**

1. A mixture of a substance having thrombolytic activity and of an antithrombotic substance, wherein the substance having thrombolytic activity is t-PA arid the antithrombotic substance is hirudin.

2. A mixture as claimed in claim 1, wherein the ratio of t-PA to hirudin is from 10: 1 to 500: 1.

**Claims for the following Contracting State : ES**

1. A process for preparing an active substance solution, wherein t-PA and hirudin in a ratio of from 10:1 to 500:1 are dissolved in a buffer at pH 3.5-7 and the solution is adjusted to isotonicity.

**Revendications**

**Revendications pour les Etats contractants suivants : AT, BE, CH, DE, FR, GB, IT, LI, NL**

1. Mélange d'une substance à action thrombolytique et d'une substance antithrombotique caractérisé par le fait que la substance à action thrombolytique est t-PA et la substance antithrombotique est l'hirudine.

2. Mélange selon la revendication 1, caractérisé par le fait que le rapport du t-PA à l'hirudine est 10/1 à 500/1.

**Revendications pour l'Etat contractant suivant : ES**

1. Procédé de préparation d'une solution de produit actif, caractérisé par le fait que l'on dissout du t-PA et de l'hirudine en rapport pondéral de 10/1 à 500/1 dans un tampon au pH compris entre 3,5 et 7 et on règle la solution isotoniquement.